# EUROPÄISCHE PATENTANMELDUNG

(11) **EP 0 761 223 A1**
(43) Veröffentlichungstag der Anmeldung: **12.03.1997**
(21) Anmeldenummer: 96112849.3
(22) Anmeldetag: 09.08.1996
(51) Int. Cl.: A61K 31/505

(54) **Verwendung von Orotsäure zur Herstellung eines Arzneimittels zur Behandlung von Patienten mit schwerer Herzinsuffizienz mit Indikation zur Herztransplantation**

(30) Priorität: 17.08.1995 DE 19530319
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Verwendung von Orotsäure zur Behandlung von Patienten mit Indikation zur Herztransplantation. Orotsäure wird dabei insbesondere zur Reduktion der Herz-Kreislauf-Mortalität verwendet.

## Beschreibung

Die vorliegende Erfindung betrifft ein Produkt zur Behandlung von Patienten mit Indikation zur Herztransplantation.

Für Patienten mit schwerer austherapierter Herzinsuffizienz existiert im Endeffekt nur die Möglichkeit einer Herztransplantation. Dieser schwere Eingriff kann jedoch nur Patienten zugemutet werden, die auch die Chance haben, diesen Eingriff zu überleben, nicht jedoch multimorbiden präfinalen Patienten oder Patienten in höherem Lebensalter.

Wenn bei einem Patienten die Indikation zur Herztransplantation gestellt worden ist, wird der Patient bei einer der wenigen Kliniken angemeldet, in der Herztransplantationen durchgeführt werden können, wozu der Patient dort auf eine Warteliste gesetzt wird.

Da gegenwärtig die Nachfrage nach Herztransplantationen größer ist als die OP-Kapazität der einschlägigen Kliniken, vergehen teilweise viele Monate bis die Herztransplantation dann tatsächlich durchgeführt werden kann.

Da die betreffenden Patienten alle schwer herzkrank sind, z. B. eine Koronare Herzkrankheit, Herzinsuffizienz oder Klappenfehler aufweisen, ist die Mortalität dieser Patienten besonders hoch.

Bis zur tatsächlich erfolgenden Herztransplantation werden diese Patienten hauptsächlich durch Kardiaka, wie antianginöse Therapeutika, Antihypertonika, Antiarrhythmika, positiv inotrope Substanzen (z. B. Digitalis), Diuretika, ACE-Inhibitoren, Sauerstoff etc. behandelt. Viele dieser Behandlungen bringen unerwünschte Nebenwirkungen mit sich, die unter Umständen die Mortalität dieser Patientengruppe sogar noch steigern.

Vor diesem Hintergrund ist es Aufgabe der vorliegenden Erfindung, ein Produkt zur Behandlung von Patienten mit Indikation zur Herztransplantation bereitzustellen, durch das die Mortalität gesenkt werden kann.

Erfindungsgemäß wird diese Aufgabe dadurch gelöst, daß Orotsäure zur Behandlung von Patienten mit Indikation zur Herztransplantation verwendet wird.

Orotsäure wird bisher in Salzform als sogenannter Mineral- oder Elektrolyt-Schlepper eingesetzt und dient dazu, bestimmte Kationen in die Zelle zu transportieren. In diesem Zusammenhang ist es aus der DE-OS-24 10 181 beispielsweise bekannt, Lithiumorotat zu verwenden, um Natrium, das bei der Regulierung des Wasserhaushaltes eine Rolle spielt, in der Zelle durch Lithium zu ersetzen. Lithium wird hier in Form des Salzes der Orotsäure verwendet, da dieses den Transport des Lithiums in die Zelle bewirken kann.

Durch die Deplazierung des Natriums durch Lithium wird die Menge an gespeichertem Wasser reduziert. Dieser Effekt wird zur Therapie von Hypertonie, Arteriosklerose, Gefäß- und Gewebserkrankungen insbesondere im zerebralen Bereich sowie zur Dauer- und Schutzbehandlung von Migräne ausgenutzt.

Aus der Deutschen Apothekerzeitung, Nr. 32, 10. August 1995, Seiten 52-53 ist es zwar bekannt, daß Magnesiumorotsäure gegen Streß und Leistungsschwäche wirkt und auch eine herzschützende Wirkung postuliert werden kann, daraus kann aber keine Aussage darüber gemacht werden, wie Patienten mit schwerer Herzinsuffizienz mit einer Indikation zur Herztransplantation behandelt werden können.

Es wurde nämlich bei einem solchen schwerstkranken älteren Patienten festgestellt, daß sich nach Behandlung mit Magnesiumorotat der klinische Zustand wesentlich verbessert hat. Dieser Patient konnte sogar das Bett verlassen und sich allmählich wieder den gewohnten Betätigungen widmen. Dieser Patient fühlt sich auch noch zum Zeitpunkt der Einreichung dieser Anmeldung unter anhaltender zusätzlicher Applikation von Magnesiumorotat wohl und mußte sich daher auch keiner Herztransplantation mehr unterziehen.

Die Ursache für diese vergleichsweise schnelle und umfassende Besserung ist unbekannt und kann keineswegs nur auf blutdrucksenkender, lipidsenkender und herzentlastender Wirksamkeit von Orotsäure beruhen.

Kasuistiken mit Patienten zur Herztransplantation zeigen, daß durch Magnesiumorotat-Gabe verhindert wird, daß derartige Patienten vor der Herztransplantation versterben, wobei in einigen besonders günstigen Fällen überraschenderweise sogar eine bereits terminierte Herztransplantation unnötig wurde, wie Untersuchungen unmittelbar vor der geplanten Herztransplantation zeigten.

Weitere Untersuchungen und Studien, die in den nachstehend aufgeführten Beispielen näher besprochen werden, konnten zeigen, daß Orotsäure oder Magnesiumorotat eine positive Wirkung auf viele Faktoren und Begleiterkrankungen haben, die die Mortalität von Patienten mit Indikation zur Herztransplantation vergrößern, wenn sie nicht behandelt werden.

In weiteren Studien konnte gezeigt werden, daß Orotsäure für sich genommen und insbesondere Magnesiumorotat den Membrangradienten aufrechterhält, arteriosklerotischen Gefäßveränderungen entgegenwirkt und blutdrucksenkend wirkt. In diesem Zusammenhang konnte in Untersuchungen an Triathleten gezeigt werden, daß nach einer Magnesiumorotat-Supplementierung der Membrangradient aufrechterhalten werden kann, was ein Anzeichen dafür ist, daß die Membranintegrität trotz der starken körperlichen Belastung während des Triathlons aufrechterhalten bleibt.

Da Orotsäure und Magnesiumorotat somit als Membran- und Endothelschutzmittel wirken, ist ihre Anwendung bei Patienten mit Indikation zur Herztransplantation besonders wichtig, da bei diesen Patienten mehr oder weniger dauerhaft Ischämien auftreten. Das damit verbundene Auslecken der Zellen führt durch Elektrolytverluste zu Arrhythmien, die bei diesen Patienten besonders gefährlich sind.

In weiteren Studien konnte gezeigt werden, daß Orotsäure selbst sowohl zur Prävention als auch zur Therapie des metabolischen Syndroms verwendet werden kann, da es positive Auswirkungen auf alle Erkrankungen hat, die unter dem Begriff metabolisches Syndrom zusammengefaßt werden. Zu diesen Krankheiten zählen insbesondere Bluthochdruck, Übergewicht, Diabetes mellitus sowie Fettstoffwechselstörungen, von denen angenommen wird, daß sie eine gemeinsame metabolische Ursache haben.

In diesem Zusammenhang konnte in einem Tierexperiment gezeigt werden, daß Orotsäure arteriosklerotischen Gefäßveränderungen unter experimentellem Cholesterinstreß entgegenwirkt. Dabei zeigte sich, daß die Wirkung der reinen Orotsäure deutlich besser war als die von MgCl₂, so daß der Wirkeffekt bei Magnesiumorotat nicht primär auf Magnesium sondern auf die Orotsäure zurückzuführen ist. Weiterhin wurde in diesem Tierexperiment gezeigt, daß Orotsäure blutdrucksenkend wirkt.

Diese Beispiele zeigen, daß Orotsäure für sich genommen bereits die einzelnen, unter dem Begriff metabolisches Syndrom zusammengefaßten Krankheiten und Erkrankungen deutlich therapiert und hier auch präventiv wirkt. Durch die Verwendung von Magnesiumorotat können die Prävention und Therapie des metabolischen Syndroms noch einmal deutlich gesteigert werden.

Da der Gesundheitszustand von Patienten mit Indikation zur Herztransplantation durch Vorliegen des metabolischen Syndroms zusätzlich aggraviert wird, wirken sich Orotsäure und Magnesiumorotat somit indirekt positiv auf die Mortalität dieser Patienten aus.

Weiterhin konnte gezeigt werden, daß Orotsäure und Magnesiumorotat direkt an den Auslösemechanismen für Störungen der Mikrozirkulation angreifen, sie zeigen nämlich eine antiarteriosklerotische und blutdrucksenkende Wirkung und führen auf direkte Weise auch dazu, daß Mikroangiopathien ursächlich therapiert werden können.

Weil Patienten mit Indikation zur Herztransplantation häufig eine Störung der Mikrozirkulation aufweisen, können Orotsäure und Magnesiumorotat über ihren positiven Einfluß auf die Mikrozirkulation auch hier die Mortalität dieser Patienten auf indirekte Weise verbessern.

Schließlich konnte in einer Tierstudie gezeigt werden, daß Orotsäue die Resynthese des Energieträgers ATP stimuliert. Die Erfinder haben nun gefunden, daß dadurch nicht nur der intrazelluläre Erhalt an ATP, sondern überraschenderweise auch die Zahl der Bindungsmöglichkeiten für Magnesium erhöht wird. Damit ist die Voraussetzung dafür gegeben, daß auch das in Form vom Magnesiumorotat oder einer anderen Magnesiumverbindung zugeführte Magnesium in der Zelle in Form des Magnesium-ATP-Komplexes fixiert wird. Dies hat zur Folge, daß über die Erhöhung des Orotsäure-induzierten ATP-Gehaltes auch der intrazelluläre Magnesiumspiegel erhöht wird.

Dies ist nun für Patienten mit Indikation zur Herztransplantation besonders vorteilhaft, weil bei ihnen häufig die Magnesiumspiegel erniedrigt sind. Dies beruht insbesondere darauf, daß die Einnahme der oben beschriebenen Arzneimittel, insbesondere der Diuretika zu einem Magnesiummangel führt. Ferner kommt es bei diesen Patienten krankheitsbedingt zu einer ATP-Verarmung im Myokard.

Auch auf diese indirekte Weise wirken sich Orotsäure und Magnesiumorotat günstig auf die Mortalität der genannten Patientengruppe aus.

Ferner konnte gezeigt werden, daß Orotsäure und Magnesiumorotat das Remodelling des Herzens verbessern sowie Reperfusionschäden und einem Reinfarkt vorbeugen, wodurch sich ebenfalls positive direkte Auswirkungen auf die Mortalität diese Patientengruppe ergeben, die manchmal bereits vor der Herztransplantation am Herzen operiert wurden.

Aus all diesen Ergebnissen läßt sich nun der überraschende Schluß ziehen, daß Orotsäure selbst zur Behandlung von Patienten mit Indikation zur Herztransplantation verwendet werden kann und die Herz-Kreislauf-Mortalität reduziert. Neben dem direkten Einfluß auf die Pumpleistung und den Zustand des Herzens hat die Orotsäure positive Auswirkungen auf viele Faktoren und Begleiterkrankungen, die sich ansonsten negativ auf die Mortalität auswirken würden, wenn sie nicht therapiert oder präventiv behandelt würden.

Dies ist als überraschend anzusehen und war in dieser Deutlichkeit und Ausprägung nicht zu erwarten.

In den bereits erwähnten Studien konnte teilweise weiter gezeigt werden, daß die Verwendung von Magnesiumorotat gegenüber der Verwendung von reiner Orotsäure eine noch größere präventive und therapeutische Wirkung zeigt.

In einem Ausführungsbeispiel ist es dann bevorzugt, wenn eine Mischung aus Orotsäure und einer Magnesiumverbindung verwendet wird.

Hier ist von Vorteil, daß ein synergistischer Effekt auftritt, zum einen wird die Wirkung der Orotsäure ausgenutzt, wobei zusätzlich eine Magnesiumverbindung eingesetzt wird, um auch deren Wirkung mit zu verwenden.

Die Mischung besteht dabei vorzugsweise aus einem Salz der Orotsäure und einer Magnesiumverbindung, wobei auch eine Mischung aus Orotsäure und Magnesiumorotat verwendet werden kann.

Auch hier ist von Vorteil, daß neben der reinen Orotsäure Magnesium in dem herzustellenden Arzneimittel/Präparat/Produkt zu finden ist.

Das aus Orotsäure und/oder ihren Salzen hergestellte Arzneimittel/Produkt kann dabei in allen Darreichungsformen auftreten.

Die Erfindung wird nachfolgend anhand einiger Studien und im Zusammenhang mit den Figuren näher beschrieben und erläutert.

Es zeigen:
- Fig. 1: den Vergleich des ANF-Anstieges unter Belastung bei einer Magnesiumorotat-Gruppe und einer Kontrollgruppe;
- Fig. 2: die Leistungssteigerung von Triathleten nach Magnesiumorotat-Supplementierung;
- Fig. 3: Indizes arteriosklerotischer Gefäßveränderungen unter experimentellem "Cholesterinstreß" bei Kaninchen;
- Fig. 4: die Reduktion der in Wistar-Ratten experimentell induzierten, malignen Blutdrucksteigerung durch Magnesiumorotat; und
- Fig. 5: den ATP-Gehalt in den verschiedenen Gruppen einer Tierstudie.

### Beispiel 1: Untersuchung der Wirkung von Magnesiumorotat auf die Belastbarkeit von Patienten mit Koronarer Herzkrankheit

Im Rahmen einer doppelblinden, placebokontrollierten Studie wurden 14 Patienten einer ambulanten Herz-Sportgruppe einer vierwöchigen Therapiephase unterworfen. Alle 14 Patienten wiesen echokardiographisch ein linksventrikuläres enddiastolisches Volumen (LVEDV) von mindestens 100 ml als Zeichen einer eingeschränkten linksventrikulären Funktion auf und hatten z.T. einen Myokardinfarkt erlitten.

Nach einer fahrradergometrischen Ausgangsuntersuchung wurden unter Fortsetzung der vor Studienbeginn bestandenen Medikation zusätzlich 3 x 2 Tabletten à 500 mg Magnesiumorotat bzw. in der Kontrollgruppe Placebo appliziert. Im Anschluß an diese Applikationsphase erfolgt eine Abschlußuntersuchung.

In der Verumgruppe verringerte sich das linksventrikuläre endsystolische Volumen um 25,3 ml (p = 0,016), die Ejektionsfraktion zeigte eine Zunahme von 42,6 % auf 51,7 % (p = 0,034). Ferner zeigte sich eine schwach signifikante Abnahme des LVEDV von 130,7 ml auf 103 ml (p = 0,053).

Anhand der beschrieben verbesserten Pumpleistung des Herzens konnte auf eine Verbesserung des Remodelling des Herzens geschlossen werden.

Ferner wurde noch der atriale natriuretische Faktor (ANF) bestimmt, der bei der Regelung des Blutvolumens und Salzhaushaltes mitwirkt. Der Vergleich des ANF-Anstiegs unter Belastung zwischen Verum- und Kontrollgruppe zeigte in der Abschlußuntersuchung signifikante Unterschiede (p = 0,049).

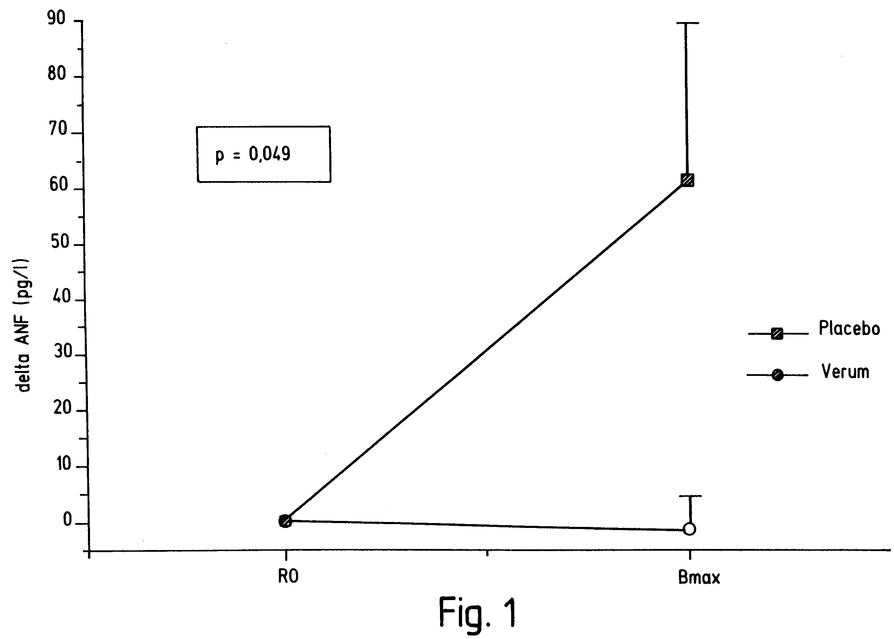

In Fig. 1 ist die Differenz des ANF-Anstieges unter Belastung (delta ANF) wiedergegeben.

Dies ist ein weiterer laborchemischer Beleg dafür, daß sich durch Magnesiumorotat der Prozeß des Remodelling gebessert hat, denn es zeigt sich, daß unter Magnesiumorotat-Gabe bei Belastung ein verminderter Füllungsdruck auftritt, was ein Zeichen einer verbesserten Myokardkontraktilität ist.

Die Verbesserung der Pumpleistung des Herzens reduziert die Mortalität von Patienten mit Indikation zur Herztransplantation auf direkte Weise.

### Beispiel 2: Untersuchung der Wirkung von Magnesiumorotat auf die Leistungsfähigkeit von Triathleten

In einer doppelblinden, randomisierten und placebokontrollierten Studie wurden 23 Triathleten einem 500 m Schwimm-, 20 km Fahrrad- und 5.000 m Lauftest nach einer vierwöchigen Magnesiumorotat-Supplementierung von 17 mmol/Tag unterworfen. Die Tests wurden ohne Pausen direkt hintereinander absolviert.

Die Schwimm- (7 min, 44 sec), Radfahr- (34 min, 54 sec) und Laufzeiten (21 min, 43 sec) lagen in der Verumgruppe durchgängig und deutlich unter denen der Kontrollgruppe (8 min, 57 sec, 35 min, 3 sec und 23 min, 27 sec). Die Schwimmzeiten waren mit p = 0,025 signifikant verschieden.

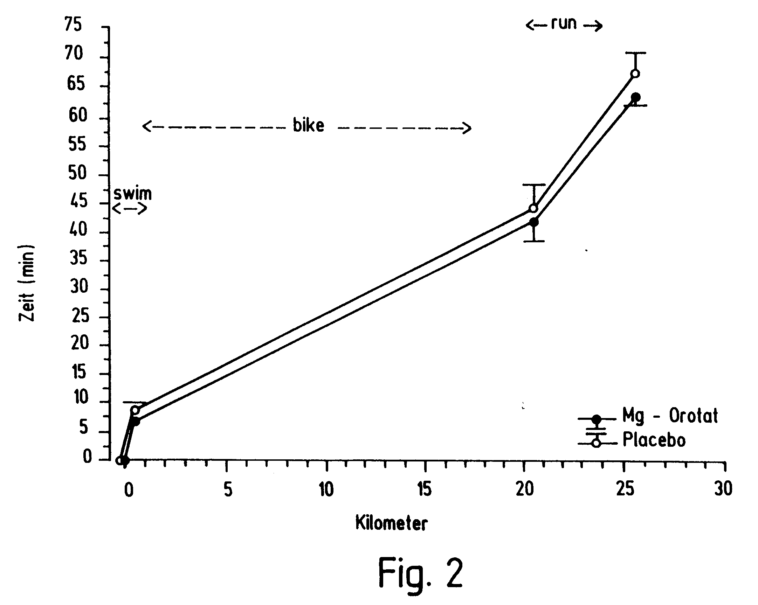

Diese Ergebnisse sind in der Fig. 2 dargestellt und zeigen eine deutlichere Verbesserung der Leistungsfähigkeit der Sportler aus der Verumgruppe.

Auch die Erhöhung der Leistungsfähigkeit wirkt sich direkt positiv auf die Mortalität der hier interessierenden Patienten aus.

### Beispiel 3: Untersuchung der Wirkung von Magnesiumorotat auf den Membranstoffwechsel von Triathleten

In einer doppelblinden, randomisierten und placebokontrollierten Studie wurden 23 Triathleten einem 500 m Schwimm-, 20 km Fahrrad- und 5.000 m Lauftest nach einer vierwöchigen Magnesiumorotat-Supplementierung von 17 mmol/Tag unterworfen. Die Tests wurden ohne Pausen direkt hintereinander absolviert. Vor dem Test, zwischen den Disziplinen und am Ende wurde den Probanden Blut entnommen und auf Parameter des Membranstoffwechsels untersucht.

Zur Untersuchung der Membran wurde jetzt die Konzentration von Creatinkinase im Blut untersucht. Creatinkinase ist eines der Enzyme, das die Zelle bei Schädigung der Membran verliert, so daß es für die Messung der Membranintegrität als Leitenzym verwendet werden kann. Mit anderen Worten, die Anwesenheit von Creatinkinase und Creatinin im Blut deutet auf Membranschädigungen hin.

In dieser Untersuchung lagen nun die streßbedingten Creatininkonzentrationen in der Verumgruppe durchweg niedriger als die der Kontrollgruppe. Die streßbedingte, relative Erhöhung der Creatininkonzentration war in der Verumgruppe mit 124 ± 18 % im Vergleich zur Placebogruppe mit 138 ± 15 % mit p = 0,07 fast statistisch signifikant niedriger.

Der relative, streßbedingte Anstieg der Creatinkinase-Aktivität im Serum fiel in der Kontrollgruppe mit 41 % signifikant höher aus als in der Verumgruppe mit 22 %.

Die verminderte streßbedingte Freisetzung der Creatinkinase durch Magnesiumorotat-Supplementierung deutet auf eine verbesserte Aufrechterhaltung der Membrangradienten während des Triathlons in der Verumgruppe hin, so daß bei Patienten mit Indikation zur Herztransplantation die Gefahr von Arrhythmien gemindert wird.

### Beispiel 4: Untersuchung der Wirkung von Orotsäure und Magnesiumorotat in einem Arteriosklerose-Modell

In einer Versuchsreihe mit einem Arteriosklerose-Modell wurden Kaninchen über 112 Tage einer zweiprozentigen Cholesterindiät ausgesetzt und mit äquimolaren Mengen von Magnesiumchlorid, Orotsäure und Magnesiumorotat bzw. in der Kontrollgruppe überhaupt nicht weiter behandelt. Zusätzlich zu histologischen Untersuchungen der Koronaarterien sowie der Aorta femoralis und renalis wurde der Zustand der Aorta an jeweils mehreren Stellen auch planimetrisch-photographisch dokumentiert. Mittels einer computergestützten, digitalen Methode der Oberflächenmessung ("density slice"-Methode) erhält man hierbei dreidimensionale Bilder, mit deren Hilfe auch eine Quantifizierung der cholesterininduzierten Lumeneinengungen möglich ist.

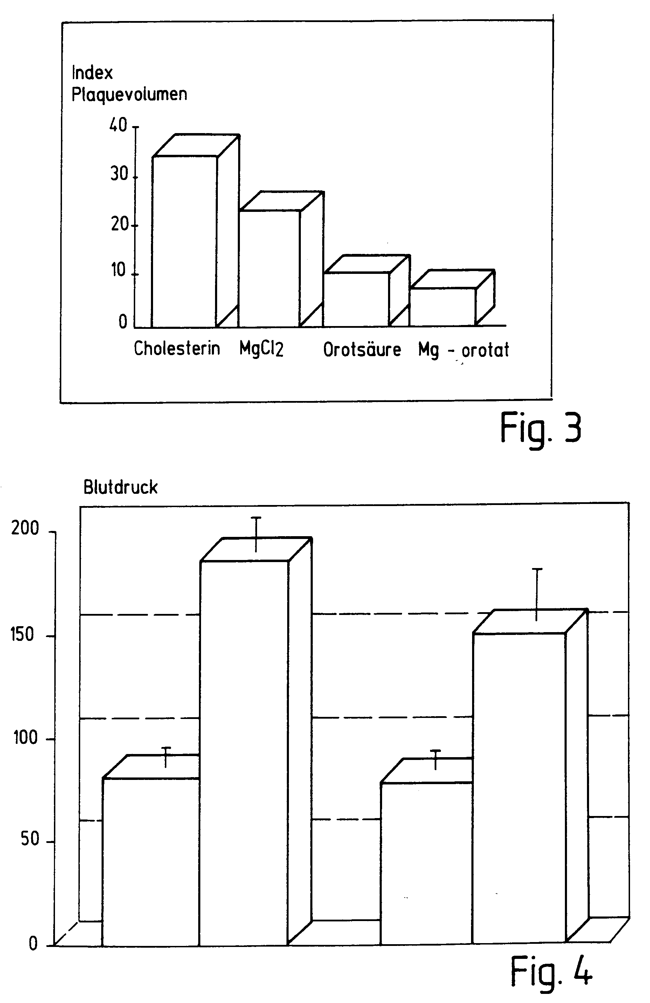

In Fig. 3 ist das Ergebnis dieser Untersuchung dargestellt. Es ist zu erkennen, daß die Gefäßverengungen in der unbehandelten Kontrollgruppe mit einem Index von 34,36 am ausgeprägtesten waren. Die Orotsäure wirkt mit einem Index von 9,77 deutlich besser den arteriosklerotischen Läsionen entgegen als Magnesiumchlorid (Index 22,63).

Als bester Schutz erwies sich jedoch Magnesiumorotat mit einem Index von 7,28.

Diese quantifizierten Ergebnisse wurden durch die licht- bzw. elektronenmikroskopischen Befunde sowie durch enzymhistochemische Untersuchungen bestätigt. Demnach verhütet Magnesiumorotat auch in den Koronarien die Genese sklerotischer Läsionen am besten. Die lumeneinengende, durch die Cholesterinfütterung induzierte Proliferation von Schaumzellen war unter dieser Medikation weitaus am geringsten ausgeprägt.

Aus dieser Untersuchung ergibt sich, daß Orotsäure selbst arteriosklerotische Gefäßveränderungen entscheidend verhütet und daß diese Wirkung durch Zugabe von Magnesium weiter gesteigert werden kann.

### Beispiel 5: Untersuchung der Wirkung von Magnesiumorotat im Hypertonie-Tiermodell

In den Hypertonie-Tiermodellen nach Rojo-Ortega-Genest (ROG-Modell) und Lörincz-Goracz (LG-Modell) wird bei Wistar-Ratten die Aorta zwischen den beiden Renalarterien unterbunden bzw. die Niere in eine Gummimanschette fest eingepreßt, was einen malignen Hypertonus hervorruft, also eine Hypertonie mit konstanter Erhöhung des arteriellen diastolischen Blutdruckes auf > 120 mmHg.

Unter täglicher Zufuhr von 300 mg Magnesiumorotat waren weit weniger periarterielle Zellinfiltrationen und Herzmuskelzellausfälle zu beobachten als ohne Behandlung.

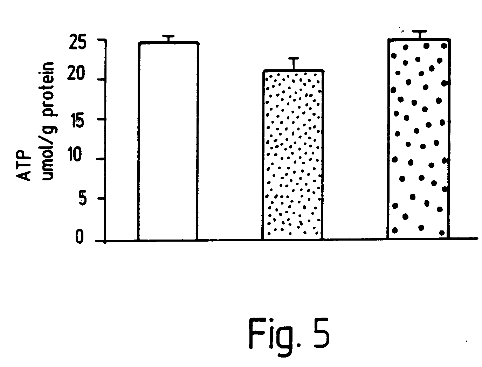

Das Ergebnis dieser Untersuchung ist in Fig. 4 dargestellt, wobei die dunklen Säulen jeweils den Beginn der ROG-Hypertonie anzeigen, während die hellen Säulen das Ende der ROG-Hypertonie darstellen. Links in Fig. 4 ist die ROG-Hypertonie ohne Behandlung gezeigt, während die beiden rechten Säulen den Verlauf der ROG-Hypertonie unter Zugabe von täglich 300 mg Magnesiumorotat wiedergibt.

Diese Therapie reduzierte die experimentiell induzierte, maligne Blutdrucksteigerung per se. Der Wirkeffekt des Magnesiumorotats ergibt sich dabei aus der Differenz zwischen den beiden hellen Säulen. Der Blutdruck betrug im ROG-Modell 152 mmHg systolisch gegenüber 186 mmHg bei unbehandelten Kontrolltieren nach Ausgangswerten von 79 bzw. 81 mmHg, war also bei den behandelten Tieren deutlich geringer ausgeprägt.

### Beispiel 6: Untersuchung der Wirkung von Magnesiumorotat auf den Energiestoffwechsel von Triathleten

In einer doppelblinden, randomisierten und placebokontrollierten Studie wurden 23 Triathleten einem 500 m Schwimm-, 20 km Fahrrad- und 5.000 m Lauftest nach einer vierwöchigen Magnesiumorotat-Supplementierung von 17 mmol/Tag unterworfen. Die Tests wurden ohne Pausen direkt hintereinander absolviert. Vor dem Test, zwischen den Disziplinen und am Ende wurde den Probanden Blut entnommen und auf Parameter des Energiestoffwechsels untersucht.

Vor dem Triathlon-Test waren die Glukosekonzentrationen beider Gruppen vergleichbar. Während des Tests stieg die Glukosekonzentration der Kontrollgruppe um 187 ± 34 %, die der Verumgruppe signifikant niedriger um 118 ± 45 % an. Gleichzeitig erhöhte sich die Insulinkonzentration der Kontrollgruppe durch den Streß auf 139 ± 101 %, in der Verumgruppe fiel die Insulinkonzentration im Vergleich zur Kontrolle dagegen signifikant auf 65 ± 43 % der Anfangswerte ab.

Diese streßabhängigen Veränderungen des Energiestoffwechsels deuten darauf hin, daß nach einer Magnesiumorotat-Supplementierung die Glukose aufgrund der besseren Insulinnutzung besser verwertet wird.

### Beispiel 7: Koronarangiographische Untersuchungen zu Beispiel 4

Zusätzlich zu den planimetrisch-photographischen Untersuchungen wurde bei der Versuchsreihe aus Beispiel 4 auch eine koronarangiographische Untersuchung vorgenommen.

Es zeigte sich, daß der experimentell hervorgerufene Cholesterinstreß in der unbehandelten Kontrollgruppe zu Mikroangiopathien in Koronarien führt.

In der Verumgruppe, die mit Orotsäure bzw. Magnesiumorotat behandelt wurde, ergab sich eine präventive und therapeutische Wirkung bei Mikroangiopathien von kleinen und kleinsten Blutgefäßen am Herzen, was sich positiv auf die Mortalität von Patienten mit Indikation zur Herztransplantation auswirkt.

### Beispiel 8: Untersuchung des ATP-Gehaltes nach einem mittleren Infarkt

Um den Einfluß von Orotsäure auf die Mortalitätsrate bei kardialen Operationen zu untersuchen, wurde eine placebokontrollierte tierexperimentelle Studie mit 29 Greyhounds durchgeführt.

Den Tieren wurde die linke vordere Koronaarterie abgeklemmt, so daß ein mittlerer Infarkt (ca. 27 %) entstand. Nach vier Tagen Erholungszeit wurde sowohl bei der mit Orotsäure behandelten Gruppe als auch bei der Kontrollgruppe eine Herzoperation mit einer Stunde Herzstillstand bei 28°C simuliert.

Zur Bestimmung biochemischer Parameter wurde dann am Myokard eine Biopsie durchgeführt. Die Biopsie ergab eine Normalisierung des ATP-Spiegels bei der Verumgruppe, während in der Kontrollgruppe der ATP-Spiegel signifikant niedriger blieb als bei Tieren ohne Infarkt.

In Fig. 5 sind die Ergebnisse dieser Studie in Form eines Balkendiagrammes dargestellt. Die linke Säule zeigt Tiere ohne Infarkt, die mittlere Säule zeigt Tiere mit Infarkt, aber ohne Orotsäurebehandlung, während die rechte Säule Ergebnisse für Tiere mit Infarkt zeigt, die danach mit Orotsäure behandelt wurden. Der Figur ist zu entnehmen, daß durch die Orotsäure-Behandlung der ATP-Spiegel wieder auf den Normalwert angsteigt.

Aus dieser Tierstudie ergibt sich, daß die Orotsäure in der Lage ist, den ATP-Gehalt in der Zelle zu erhöhen. Damit ist die Voraussetzung dafür gegeben, daß auch das in Form von Magnesiumorotat oder einer anderen Magnesiumverbindung zugeführte Magnesium in der Zelle in Form des Magnesium-ATP-Komplexes fixiert werden kann. Dies hat zur Folge, daß über die Erhöhung des Orotsäure-induzierten ATP-Gehaltes auch der intrazelluläre Magnesiumspiegel erhöht wird.

Die obigen Beispiele 1, 2 und 7 zeigen, daß Orotsäure und Magnesiumorotat sich direkt positiv auf die Funktion des Herzens auswirken, wodurch die Mortalität von Patienten mit Indikation zur Herztransplantation direkt verbessert wird.

Aus den übrigen Beispielen läßt sich erkennen, daß viele Faktoren und Begleiterkrankungen, die sich ansonsten negativ auf die Mortalität dieser Patienten auswirken würden, durch eine Medikamentierung mit Orotsäure und insbesondere mit Magnesiumorotat positiv beeinflußt werden, so daß hierdurch die Mortalität in dieser Patientengruppe indirekt positiv beeinflußt wird.

Orotsäure und Magnesiumorotat erhalten den Membrangradient aufrecht, wirken antiarteriosklerotisch und blutdrucksenkend und führen zu einer besseren Insulinausnutzung, so daß alle Krankheiten und Erkrankungen positiv beeinflußt werden, die unter dem Begriff metabolisches Syndrom zusammengefaßt werden, das den Zustand der hier interessierenden Patientengruppe negativ beeinträchtigt.

Auch über die Erhöhung des intrazellulären Magnesiumspiegels wird die Mortalität dieser Patienten gesenkt.

## Patentansprüche

1. Verwendung von Orotsäure zur Herstellung eines Arzneimittels zur Behandlung von Patienten mit schwerer Herzinsuffizienz mit Indikation zur Herztransplantation.

2. Verwendung nach Anspruch 1, dadurch gekennzeichnet, daß Orotsäure zur Reduktion der Herz-Kreislauf-Mortalität solcher Patienten verwendet wird.

3. Verwendung nach Anspruch 1 oder Anspruch 2, dadurch gekennzeichnet, daß ein Salz der Orotsäure verwendet wird.

4. Verwendung nach Anspruch 3, dadurch gekennzeichnet, daß Magnesiumorotat verwendet wird.

5. Verwendung nach einem der Ansprüche 1 bis 4, dadurch gekennzeichnet, daß eine Mischung aus Orotsäure und einer Magnesiumverbindung verwendet wird.

6. Verwendung nach einem der Ansprüche 1 bis 5, dadurch gekennzeichnet, daß eine Mischung aus einem Salz der Orotsäure und einer Magnesiumverbindung verwendet wird.

7. Verwendung nach Anspruch 5 oder 6, dadurch gekennzeichnet, daß eine Mischung aus Orotsäure und Magnesiumorotat verwendet wird.
